# EUROPÄISCHE PATENTSCHRIFT

(11) **EP 2 198 894 B1**
(45) Veröffentlichungstag und Bekanntmachung des Hinweises auf die Patenterteilung: **15.03.2017**
(21) Anmeldenummer: 09014825.5
(22) Anmeldetag: 30.11.2009
(51) Int. Cl.: A61L 27/16, A61L 27/44, A61L 27/50

(54) **Polymethylmethacrylat-Knochenzement-Zusammensetzung zur kontrollierten Hyperthermiebehandlung**
Polymethylmethacrylate-bone cement compound for controlled hyperthermia treatment
Composition de ciment osseux en poly-méthyle-méthacrylate destinée à la manipulation hyperthermique contrôlée

(30) Priorität: 22.12.2008 DE 102008064036
(43) Veröffentlichungstag der Anmeldung: 23.06.2010
(73) Patentinhaber: Heraeus Medical GmbH, 61273 Wehrheim (DE)
(72) Erfinder: Vogt, Sebastian, Dr., 99092 Erfurt (DE); Büchner, Hubert, Dr., 64354 Reinheim (DE)
(74) Vertreter: Heraeus IP

(56) Entgegenhaltungen:
- WO-A1-2006/125452
- WO-A2-2008/073190
- WO-A2-2008/074804
- US-A- 5 411 730

## Beschreibung

Gegenstand der Erfindung ist ein durch magnetische Wechselfelder erwärmbarer Polymethylmethacrylat-Knochenzement zur kontrollierten Hyperthermiebehandlung von Knochenmetastasen.

Vielfach sind Krebserkrankungen der Prostata, der Zervix und der Brust mit der Bildung von Metastasen im Skelettsystem verbunden. Insbesondere im fortgeschrittenen, finalen Stadium der Krebserkrankung stellen Knochenmetastasen ein sehr ernstes Problem dar. Knochenmetastasen sind für den Patienten mit zum Teil extremen Schmerzen verbunden. Weiterhin führen Metastasen in der Wirbelsäule durch Zerstörung der Knochensubstanz der Wirbel zu Kompressionsbrüchen. Bei dem Zusammenfallen der Wirbel kommt es zu Kompressionen des Wirbelkanals und damit des Rückenmarks. Die Folge davon sind Lähmungserscheinungen und extreme Schmerzen. Es wird deshalb vielfach als palliative Maßnahme für die letzten Lebensmonate der Patienten versucht, durch Auffüllung der zerstörten Wirbel mit Knochenzement eine Kompression des Rückenmarks zu vermindern bzw. zu verhindern. Damit wird häufig eine Verbesserung der Lebensqualität der Patienten erreicht. Der für die Auffüllung genutzte Knochenzement muss daher nur eine geringe mechanische Festigkeit aufweisen.

Es hat in der Vergangenheit Versuche gegeben, den zur Auffüllung der zerstörten Wirbelkörper eingesetzten Knochenzement mit cytostatischen Wirkstoffen zu dotieren, damit durch lokale Freisetzung der cytostatischen Wirkstoffe ein weiteres Wachstum der Metastasen vermindert oder herausgezögert wird (DE 35 13 938). Problematisch ist dabei die hohe Toxizität der Cytostatika, insbesondere von Methotrexat. Bei zu hoher Dosierung treten leicht unerwünschte Nebenwirkungen auf. Die Dosierung von Cytostatika im Zement ist daher sehr problematisch.

Im Rahmen der Therapie von soliden Tumoren fand in den letzten Jahren die Hyperthermie verstärktes Interesse. Neben der kontrovers diskutierten Ganzkörperhyperthermie wurde insbesondere die lokale Hyperthermie als Therapiemethode näher untersucht. Dabei wird Tumorgewebe auf Temperaturen im Bereich von 40-46 °C erwärmt. Die lokale Überhitzung des Tumorgewebes soll eine Nekrose des Tumors induzieren oder die Empfindlichkeit des Tumorgewebes gegenüber Cytotstatika oder einer Strahlentherapie infolge einer thermischen Vorschädigung der Tumorzellen erhöhen. Neben konventionellen Methoden der lokalen Erwärmung mit Mikrowellen, Ultraschall und magnetischer "Thermoseeds", wurden insbesondere Entwicklungen zur Nutzung von magnetischen/superparamagnetischen Partikeln, insbesondere Nanopartikeln durchgeführt. Diese Partikel erwärmen sich bei Einwirkung von magnetischen Wechselfeldern. Exemplarisch für diese Entwicklungen stehen die Patentschriften EP 1 952 825, CN101219071, WO2008074804, US 2006/147381, EP 1 883 425, WO2007142674, US 2005/249817 und US 6,541,039. Besonders interessant ist dabei US 2005/249817. Hierbei werden für die Hyperthermiebehandlung magnetische Nanopartikel vorgeschlagen, die eine Curie-Temperatur von 40-46 °C haben. Oberhalb der Curie-Temperatur verhalten sich die vorgeschlagenen Partikel nur noch paramagnetisch und damit wird der Energieeintrag in das Tumorgewebe begrenzt. Es soll dadurch eine Temperaturregulation erreicht werden, um eine nicht notwendige Überwärmung des Gewebes über 46°C zu verhindern. Temperaturen von ca. 43 °C sind als ausreichend anzusehen, um das Tumorgewebe zu schädigen bzw. zur Nekrose zu bringen. Eine Erwärmung auf höhere Temperaturen bringt keinen zusätzlichen Vorteil und ist mit der Gefahr einer thermischen Schädigung des umliegenden gesunden Gewebes verbunden.

Grundsätzlich können übliche Polymethylmethacrylat-Knochenzemente zur Auffüllung von Knochenkavitäten verwendet werden, die durch Knochenmetastasen entstanden sind. Polymethylmethacrylat-Knochenzemente haben bisher einen grundsätzlich gleichen Aufbau. Polymethylmethacrylat-Knochenzemente sind seit Jahrzehnten bekannt und klinisch bewährt. Sie gehen auf die grundlegenden Arbeiten von Sir Charnley zurück (Charnley, J.: Anchorage of the femoral head prosthesis of the shaft of the femur. J. Bone Joint Surg. 42 (1960) 28-30.). Der Grundaufbau der Polymethylmethacrylat-Knochenzemente ist seit dem prinzipiell gleich geblieben. Polymethylmethacrylat-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente besteht aus einem oder mehreren Polymeren, die auf Basis von Methylmethacrylat und Comonomeren, wie Styren, Methylacrylat oder ähnlichen Monomeren durch Polymerisation, vorzugsweise Suspensionspolymerisation, hergestellt sind, einem Röntgenopaker und dem Initiator Dibenzoylperoxid. Beim Vermischen der Pulverkomponente mit der Monomerkomponente entsteht durch Quellung der Polymere der Pulverkomponente im Methylmethacrylat ein plastisch verformbarer Teig. Gleichzeitig reagiert der Aktivator N,N-Dimethyl-p-toluidin mit Dibenzoylperoxid, dass unter Bildung von Radikalen zerfällt. Die gebildeten Radikale initiieren die radikalische Polymerisation des Methylmethacrylates. Mit fortschreitender Polymerisation des Methylmethacrylates erhöht sich die Viskosität des Zementteigs bis der Zementteig erstarrt und damit ausgehärtet ist.

Durch magnetische Wechselfelder erwärmbare Monomermischungen für Knochenzemente sind beispielswese in der Europäischen Patentanmeldung 08 017 156.4 vorgeschlagen worden. Die Erwärmung aktiviert thermisch zerfallende Initiatoren und lässt das Material aushärten.

WO2006/125452 offenbart die Verwendung von superparamagnetischen Eisen-Nanopartikeln für die Hyperthermie-Behandlung. Diese Nanopartikel können mit einem biokompatiblen Polymer beschichtet sein. Zweck dieser Beschichtung ist es die Biokompatibilität der Eisen-Nanopartikel zu verbessern.

Der Erfindung liegt die Aufgabe zu Grunde, einen Polymethylmethacrylat-Knochenzement zu entwickeln, der eine Auffüllung von durch Knochenmetastasen verursachten Knochenkavitäten ermöglicht. Der Polymethylmethacrylat-Knochenzement soll durch Einwirkung von magnetischen Wechselfeldern so erwärmt werden können, dass eine gezielte, lokale thermische Schädigung von nachwachsendem Tumorgewebe möglich ist. Es sollen dabei jedoch unerwünschte, temporäre Temperaturspitzen sicher verhindert werden, um die Gefahr einer thermischen Schädigung des umgebenden gesunden Gewebes einzuschränken, die zu Schmerzen und weiteren unerwünschten Folgen für den Patienten führen könnte.

Die Aufgabe wird dadurch gelöst, dass ein Polymethylmethacrylat-Zement zur kontrollierten Hyperhermiebehandlung, mit den üblichen Komponenten Polymethylmethäcrylat, Röntgenopaker, Methylmethacrylat und/oder Alkylmethacrylaten sowie Initiatorsystem bereitgestellt wird, der ferromagnetische und/oder superparamagnetische Partikel mit einer Partikelgröße bis maximal 500 µm und mindestens eine biodegradierbare oder biokompatible, kristalline Substanz mit einem Schmelzpunkt im Bereich von 42-80°C enthält. Die Erfindung betrifft mithin Polymethylmethacrylat-Knochenzement-Zusammensetzungen zur kontrollierten Hyperthermiebehandlung, enthaltend
A Polymethylmethacrylat,
B Röntgenopaker,
C Methylmethacrylat und/oder Alkylmethacrylaten,
D Initiatorsystem,
E ferromagnetische und/oder superparamagnetische Partikel mit einer Partikelgröße bis maximal 500 µm sowie
F mindestens eine biodegradierbare oder biokompatible, kristalline Substanz mit einem Schmelzpunkt im Bereich von 42-80°C.

Durch Einwirkung von magnetischen Wechselfeldern auf die im Zement enthaltenen ferromagnetischen und/oder superparamagnetischen Partikel kann der ausgehärtete Polymethyl-meth,acrylat-Zement *in vivo* erwärmt werden. Die im Zement enthaltene biodegradierbare oder biokompatible, kristalline Substanz mit einem Schmelzpunkt im Bereich von 42-80°C wird ebenfalls erwärmt. Proportional zum Energieeintrag steigt die Temperatur des Zementes, bis der Schmelzpunkt der kristallinen Substanz erreicht ist. Dann verbleibt die Temperatur des Zementes so lange bei der Schmelztemperatur, bis die kristalline Substanz vollständig geschmolzen ist. Die zugeführte Energie wird als latente Energie gespeichert. Temperaturspitzen werden dadurch sicher abgefangen und eine unerwünschte Überwärmung des Gewebes und angrenzender Körperregionen wird vermieden.

Vorzugsweise sind die ferromagnetischen Partikel von der kristallinen Substanz teilweise oder vollständig umhüllt. Eisen, Kobalt, Samarium-Cobalt, Neodym-Eisen (Nd₂Fe₁₄) sind als ferromagnetische Partikel bevorzugt. Als superparamagnetische Partikel dient bevorzugt Magnetit.

Als biodegradierbare, kristalline Substanzen kommen 1,2,3-Trilauroylglycerin, 1,2,3-Trimyristylglycerin, 1,2,3-Tripalmitoylglycerin, Laurinsäure, Myristinsäure und Palmitinsäure in Frage. Besonders bevorzugt ist 1,2,3-Trilauroylglycerin mit einer Schmelztemperatur von 43 °C. Die biodegradierbare Substanz ist zweckmäßigerweise bis zu einem Gehalt von 30 Gewichtsprozent im Polymethylmethacrylat-Knochenzement enthalten. Dadurch wird ein sicheres Abfangen von Temperaturspitzen gewährleistet.

Die erfindungsgemäße Knochenzement-Zusammensetzung kann zusätzlich Cytostatika enthalten. Geeignete sind dem Fachmann für die betreffende Indikation bekannt. Bevorzugt ist dabei als Standard das Cytostatikum Methotrexat.

Erfindungsgemäße Zusammensetzungen eignen sich zur Herstellung von Mitteln zum zeitweisen Überwärmen von Körperregionen (Hyperthermie) ohne unerwünschte Temperaturspitzen, besonders des Bereichs der Wirbelsäule, vorzugsweise auf 43-46°C.

Insbesondere sind die erfindungsgemäßen Zusammensetzungen für die palliative Auffüllung und Stabilisierung von durch Knochenmetastasen gebildeten Knochenkavitäten bestimmt, wobei der Zement eine gezielte lokale Hyperthermiebehandlung ermöglichen soll, um eventuell nachwachsendes Tumorgewebe lokal palliativ behandeln zu können. Der Polymethacrylat-Knochenzement soll auf diese Weise die Lebensqualität von Tumorpatienten verbessern helfen.

Die Erfindung wird durch nachstehende Beispiele erläutert, ohne jedoch die Erfindung zu beschränken. Teile- und Prozentangaben beziehen sich - wie in der übrigen Beschreibung - auf das Gewicht, sofern nicht anders angegeben.

### Beispiel 1

### Herstellung von Zementpulver

Es wurden mit Hilfe eines Turbula-Mischers unter Verwendung von Porzellanmahlkörpern in einer Kunststoff-Flasche 30,0 g 1,2,3-Trilauroylglycerin mit 10,0 g Magnetitpartikeln (D₅₀ 100 µm) intensiv 30 Minuten vermahlen. Das 1,2,3-Trilauroylglycerin haftet dann auf der Oberfläche der Magnetitpartikel. Danach wurden 60,0 g Poly-(methylmethacrylat-co-methylacrylat) und 2,0 g Dibenzoylperoxid (phlegmatisiert mit 25 % Wasser) dazugeben. Das Gemisch wurde weitere 30 Minuten gemahlen. Das Zementpulver lag nach dem Mahlen als grau-braunes Pulver vor.

### Beispiel 2

Es wurden 40,0 g des im Beispiel 1 hergestellten Zementpulvers mit 20 ml eines Gemisch aus 18,40 g Methylmethacrylat und 0,38 g p-N,N-Dimethyl-toluidin vermischt. Es entstand innerhalb von 30 Sekunden ein homogener Zementteig, der nach wenigen Minuten aushärtete. Mit dem Zementteig wurde unter Verwendung einer Kunststoffform ein zylindrischer Formkörper (Höhe 10 mm, Durchmesser 25 mm) hergestellt, wobei ein elektronischer Temperaturfühler in die Mitte des Formkörpers positioniert wurde. Der ausgehärtete Formkörper wurde mit Hilfe einer bei konventionellen Induktionsherden entlehnten Induktionsheizung (Spule mit Steuerungselektronik, Frequenz 25 kHz) innerhalb von zwei Minuten erwärmt. Es wurde dabei eine maximale Temperatur von 43-44 °C im Inneren des Formkörpers gemessen.

## Patentansprüche

1. Polymethylmethacrylat-Knochenzement-Zusammensetzung zur kontrollierten Hyperthermiebehandlung, enthaltend
**A** Polymethylmethacrylat,
**B** Röntgenopaker,
**C** Methylmethacrylat und/oder Alkylmethacrylaten,
**D** Initiatorsystem,
**E** ferromagnetische und/oder superparamagnetische Partikel mit einer Partikelgröße bis maximal 500 µm sowie
**F** mindestens eine biodegradierbare oder biokompatible, kristalline Substanz mit einem Schmelzpunkt im Bereich von 42-80°C.

2. Polymethylmethacrylat-Knochenzement-Zusammensetzung nach Anspruch 1, **dadurch gekennzeichnet, dass** die Partikel der Komponenten E von der kristallinen Substanz **F** teilweise oder vollständig umhüllt sind.

3. Polymethylmethacrylat-Knochenzement-Zusammensetzung nach Anspruch 1 oder 2, **dadurch gekennzeichnet, dass** die Partikel der Komponente **E** aus Eisen, Kobalt, Samarium-Cobalt, Neodym-Eisen (Nd₂Fe₁₄) oder Magnetit bestehen.

4. Polymethylmethacrylat-Knochenzement-Zusammensetzung nach mindestens einem der vorstehenden Ansprüche, **dadurch gekennzeichnet, dass** die Substanz der Komponente F der Gruppe bestehend aus 1,2,3-Trilauroylglycerin, 1,2,3-Trimyristylglycerin, 1,2,3-Tripalmitoylglycerin, Laurinsäure, Myristinsäure und Palmitinsäure angehört.

5. Polymethylmethacrylat-Knochenzement-Zusammensetzung nach mindestens einem der vorstehenden Ansprüche, **dadurch gekennzeichnet, dass** die Komponente F bis zu einem Gehalt von 30 Gewichtsprozent bezogen auf die Gesamtmasse im Polymethylmethacrylat-Knochenzement enthalten ist.

6. Polymethylmethacrylat-Knochenzement-Zusammensetzung nach mindestens einem der vorstehenden Ansprüche, **dadurch gekennzeichnet, dass** zusätzlich mindestens ein Cytostatikum enthalten ist.

7. Verwendung einer Zusammensetzung nach einem der vorstehenden Ansprüche zur Herstellung eines Mittels zum zeitweisen Erwärmen von Körperregionen (Hyperthermie) ohne unerwünschte Temperaturspitzen.

8. Verwendung nach Anspruch 7 zur Herstellung eines Mittels zum zeitweisen Erwärmen (Hyperthermie) des Bereichs der Wirbelsäule ohne unerwünschte Temperaturspitzen.

9. Verwendung einer Zusammensetzung nach Anspruch 7 oder 8 zur Herstellung eines Mittels zum zeitweisen Erwärmen (Hyperthermie) von Körperregionen auf 43-46°C.

## Claims

1. Polymethylmethacrylate bone cement composition for controlled hyperthermia treatment, containing
A. polymethylmethacrylate;
B. radio-opaquer;
C. methylmethacrylate and/or alkyl methacrylates;
D. initiator system;
E. ferromagnetic and/or superparamagnetic particles having a particle size of maximally 500 µm as well as
F. at least one biodegradable or biocompatible crystalline substance having a melting point in the range of 42-80°C.

2. Polymethylmethacrylate bone cement composition according to claim 1, **characterised in that** the particles of component E are partially or completely enveloped by crystalline substance F.

3. Polymethylmethacrylate bone cement composition according to claim 1 or 2, **characterised in that** the particles of component E consist of iron, cobalt, samarium-cobalt, neodymium-iron (Nd₂Fe₁₄) or magnetite.

4. Polymethylmethacrylate bone cement composition according to at least one of the preceding claims, **characterised in that** the substance of component F belongs to the group consisting of 1,2,3-trilauroyl glycerol, 1,2,3-trimyristyl glycerol, 1,2,3-tripalmitoyl glycerol, lauric acid, myristic acid, and palmitic acid.

5. Polymethylmethacrylate bone cement composition according to at least one of the preceding claims, **characterised in that** the polymethylmethacrylate bone cement contains up to 30% by weight of component F, relative to the total mass.

6. Polymethylmethacrylate bone cement composition according to at least one of the preceding claims, **characterised in that** it additionally contains at least one cytostatic agent.

7. Use of a composition according to any one of the preceding claims for the production of an agent for temporary heating of body regions (hyperthermia) without undesired temperature peaks.

8. Use according to claim 7 for the production of an agent for temporary heating (hyperthermia) the spine without undesired temperature peaks.

9. Use of a composition according to claim 7 or 8 for the production of an agent for temporary heating of body regions (hyperthermia) to 43-46°C.

## Revendications

1. Composition de ciment osseux de polyméthylméthacrylate pour le traitement contrôlé de l'hyperthermie, contenant
A. du polyméthylméthacrylate,
B. un opacifiant radiographique,
C. du méthylméthacrylate et/ou des alkylméthacrylates,
D. un système initiateur,
E. des particules ferromagnétiques et/ou superparamagnétiques avec une taille particulaire allant jusqu'à 500 µm maximum ainsi que
F. au moins une substance cristalline biodégradable ou biocompatible avec un point de fusion dans la région de 42 à 80°C.

2. Composition de ciment osseux de polyméthylméthacrylate selon la revendication 1, **caractérisée en ce que** les particules des composants E sont partiellement ou entièrement enveloppées par la substance cristalline F.

3. Composition de ciment osseux de polyméthylméthacrylate selon la revendication 1 ou 2, **caractérisée en ce que** les particules du composant E se composent de fer, cobalt, samarium-cobalt, néodyme-fer (Nd₂Fe₁₄) ou magnétite.

4. Composition de ciment osseux de polyméthylméthacrylate selon au moins une des revendications précédentes, **caractérisée en ce que** la substance du composant F appartient au groupe constitué de la 1,2,3-trilauroylglycérine, 1,2,3-trimyristylglycérine, 1,2,3-tripalmitoylglycérine, l'acide laurique, l'acide myristique et l'acide palmitique.

5. Composition de ciment osseux de polyméthylméthacrylate selon au moins une des revendications précédentes, **caractérisée en ce que** le composant F est contenu jusqu'à une teneur de 30 pourcent en poids par rapport à la masse totale dans le ciment osseux de polyméthylméthacrylate.

6. Composition de ciment osseux de polyméthylméthacrylate selon au moins une des revendications précédentes, **caractérisée en ce qu'**au moins un cytostatique est contenu en outre.

7. Utilisation d'une composition selon une des revendications précédentes pour la fabrication d'un moyen pour le réchauffement temporaire de régions corporelles (hyperthermie) sans pics de température indésirables.

8. Utilisation selon la revendication 7 pour la fabrication d'un moyen pour le réchauffement temporaire (hyperthermie) de la région de la colonne vertébrale sans pics de température indésirables.

9. Utilisation d'une composition selon la revendication 7 ou 8 pour la fabrication d'un moyen pour le réchauffement temporaire (hyperthermie) de régions corporelles à 43 à 46°C.
